Europäisches Patentamt

European Patent Office

Office européen des brevets

(19)

(11) Publication number: **0 117 369**
**A1**

# (12) EUROPEAN PATENT APPLICATION

(21) Application number: **83308043.5**

(22) Date of filing: **30.12.83**

(51) Int. Cl.³: **A 61 L 2/24**
**// A61L2/20**

(30) Priority: **30.12.82 FI 824528**

(43) Date of publication of application: **05.09.84**
**Bulletin 84/36**

(84) Designated Contracting States: **BE DE FR GB NL**

(71) Applicant: **Orion-Yhtymä Oy Lääketehdas Orion,**
**Nilsiänkatu 10-14, SF-00510 Helsinki 51 (FI)**

(72) Inventor: **Konsi, Pekka, Sammentie 6 A,**
**02260 Espoo 26 (FI)**
Inventor: **Piskunen, Veikko, Mannikkotie 10,**
**01760 Vantaa 76 (FI)**
Inventor: **Leskela, Pekka, Kuunsirppi 2 D 137,**
**02210 Espoo 21 (FI)**

(74) Representative: **Higgins, Michael Roger et al, Marks &**
**Clerk 57/60 Lincoln's Inn Fields, London WC2A 3LS (GB)**

(54) **Process of carrying out gas sterilization and a device for use therein.**

(57) In a gas sterilisation process, a vacuum is first produced in an autoclave. The gas volume in the autoclave is then humidified by introducing steam into the gas volume of the autoclave in doses or in controlled quantity. Throughout the humidifying process a humidity transducer (13) is passed through the wall (11) of the autoclave. The transducer measures the humidity of the gas volume and on the basis of the measured value the steam supply into the gas volume is controlled. After humidification and prior to sterilization with gas the humidity transducer (13) is withdrawn to a position outside the autoclave. The transducer (13) is mounted for slidable movement in an elongate hollow support member (12, 21) which extends through the wall of the autoclave and fluid pressure operated means (29) are provided for moving the transducer between an extended and a withdrawn position.

## "PROCESS OF CARRYING OUT GAS STERILIZATION AND A DEVICE FOR USE THEREIN".

The present invention relates to a process of carrying out gas sterilization, in which process a vacuum is produced in an autoclave, whereafter the gas volume in the autoclave is humidified by introducing steam into the gas volume, and subsequent to humidifying the gas volume, sterilization of objects to be sterilized is carried out with gas in the autoclave.

The invention also relates to a device for use in a gas sterilization process.

Gas sterilization is carried out in the following way. A vacuum is first produced in the autoclave, whereafter the gas volume of the autoclave is humidified by introducing steam into the gas volume, and finally sterilization is carried out with gas. The greatest drawback of the prior art method is that the humidifying of the gas volume in the autoclave is accomplished haphazardly, and therefore the humidity rarely corresponds to the humidity value which is desired. The humidity in the autoclave gas volume may, of course, be measured by mounting a special transducer in the autoclave which also tolerates the effect of the sterilizing gas without suffering damage but such transducers are expensive.

An object of the present invention is to provide an improved process of gas sterilization and more specifically to provide a process by which gas

0117369

sterilization may be carried out in such manner that it is possible to humidify the gas volume in the autoclave in controlled manner in order to obtain reliable results.

According to a first aspect of the invention, there is provided a method of carrying out gas sterilization, in which a vacuum is produced in an autoclave, whereafter the gas volume in the autoclave is humidified by introducing steam into the gas volume, and subsequent to humidifying the gas volume sterilization with gas of objects to be sterilized is carried out in the autoclave, characterized in that the humidifying of the gas volume in the autoclave is carried out by introducing steam into the gas volume of the autoclave in doses or in controlled quantity, and throughout the humidifying process a humidity transducer is passed through the wall of the autoclave, said transducer measuring the humidity of the gas volume, and on the basis of the measured value, the steam supply into the gas volume is controlled, and in that prior to sterilization with gas the humidity transducer is taken into shelter outside the autoclave.

It is also an object of the present invention to provide a device for use in gas sterilization.

According to a second aspect of the invention, there is provided a device for use in a gas sterilization process in which a vacuum is produced in an autoclave.

and thereafter the gas volume in the autoclave is humidified by introducing steam into the gas volume, and subsequent to the humidifying of the gas volume sterilization with gas of objects to be sterilized is carried out in the autoclave, the device being characterized in that it comprises an elongate hollow support member which in use extends through the wall of an autoclave, a humidity transducer and means for moving the humidity transducer within the support member between an extended position in which in use it is passed into the autoclave and a withdrawn position, in which it is outside of the autoclave. It is thus possible to humidify the gas volume in the autoclave in a controlled fashion and with high accuracy, and in the humidity measuring and control an inexpensive commercially available humidity transducer may be employed without the need to manufacture a special and hence expensive transducer.

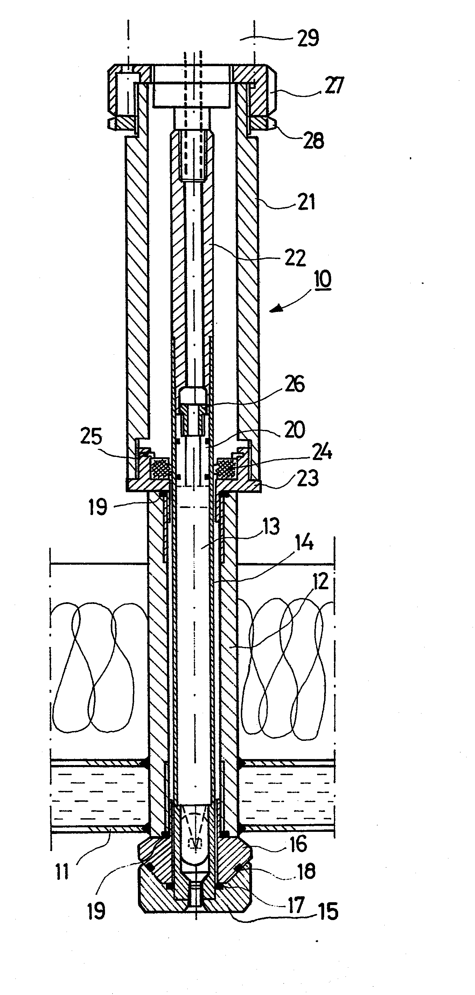

The invention will now be more particularly described by way of example, with reference to the accompanying drawing which is a cross-sectional view illustrating one embodiment of a device according to the second aspect of the invention, mounted in a wall of an autoclave and usable to perform a process according to the first aspect of the invention.

Referring to the drawing, the device is generally indicated by reference numeral 10. A humidity transducer or pick-up 13 of the device 10 is shown in a withdrawn position. The device 10 comprises the humidity transducer 13 and an elongate hollow support member comprising a wall-tube component 12 and a support tube 21. A fixing ring 27 is provided on the upper end of the support tube 21. A transducer tube 14 containing the transducer proper is slidably mounted within the wall-tube component 12. The lower end of the transducer tube 14 fits, as shown, in an end ring 15, connected in gas-tight manner through sealing flange 16, sealing rings 17 and 18 and packings 19 to the wall-tube component 12, when the transducer tube is in its withdrawn position as shown.

The reference numeral 20 indicates the upper end of the humidity transducer 13 and the reference numeral 22 indicates an extension part 22 of a piston rod (not shown), which extension part is disposed in the support tube 21. The wall-tube component 12 and the support tube 21 are mutually connected by a sealing flange 23. A packing fitted in the sealing flange 23 is shown at 24 and a retainer ring holding the packing 24 from above is shown at 25. The rounding of the tension eliminator is indicated at 26 and a clamping nut of the fixing ring 27, at 28. An hydraulic cylinder is indicated by reference numeral 29.

The operation of the device 10 will now be described in a gas sterilization process. First, the humidity transducer is extended through the wall 11 of the autoclave. Then a vacuum is produced in the autoclave. Thereafter, the gas volume in the autoclave is humidified by introducing steam, in doses or in controlled quantity, into the gas space of the autoclave. The reason for humidifying the autoclave is that dry spores are resistant to sterilizing gas. Throughout the humidifying process, the humidity transducer 13, extended into the autoclave by the piston rod of the hydraulic cylinder 29, measures the humidity of the gas volume, and on the basis of the measured value the supply of steam into the gas volume is regulated. After the desired degree of humidity has been reached, the humidity transducer 13 is retracted into shelter outside the autoclave by the piston rod of the cylinder 29. Thereafter, sterilization of objects to be sterilized is carried out in the autoclave by sterilizing gas, e.g. ethylene oxide containing gas used in conjunction with a propellant gas such as Arcton (R.T.M.) or carbon dioxide.

It is thus essential that the humidity transducer 13, which forms part of the humidity measuring and control system of the autoclave, is not exposed to the sterilizing gas during the sterilizing phase. According to the foregoing, the so-called humidifying phase can be performed before the gas sterilization proper, independent of the charge that has to be sterilized, so that the desired humidity of the gas volume is always attained in the autoclave.

6.

0117369

The component 12 and support tube 21 are formed separately rather than integrally for ease of manufacture.

The above describes a preferred embodiment of the invention and numerous modifications can be made without departing from the scope of the invention.

CLAIMS

1. A method of carrying out gas sterilization, in which a vacuum is produced in an autoclave, whereafter the gas volume in the autoclave is humidified by introducing steam into the gas volume, and subsequent to humidifying the gas volume sterilization with gas of objects to be sterilized is carried out in the autoclave, characterised in that the humidifying of the gas volume in the autoclave is carried out by introducing steam into the gas volume of the autoclave in doses or in controlled quantity, and throughout the humidifying process a humidity transducer (13) is passed through the wall (11) of the autoclave, said transducer measuring the humidity of the gas volume, and on the basis of the measured value, the steam supply into the gas volume is controlled, and in that prior to sterilization with gas the humidity transducer (13) is taken into shelter outside the autoclave.

2. A device for use in a gas sterilization process in which a vacuum is produced in an autoclave, and thereafter the gas volume in the autoclave is humidified by introducing steam into the gas volume, and subsequent to the humidifying of the gas volume sterilisation with gas of objects to be sterilized is carried out in the autoclave, the device being characterised in that it comprises an elongate hollow support member (12, 21) which in use extends through the wall (11)of an autoclave, a humidity transducer (13), and means (29) for moving the humidity transducer within the support member between an extended position in which in use it is passed into the autoclave and a withdrawn position in which it is outside of the autoclave.

3. The device of claim 2, characterised in that the support member is generally tubular and the transducer is mounted in a tube (14) which is slidably mounted in the support member.

4. The device of claim 2 or claim 3, characterised in that said means comprises fluid pressure operated means (29).

5. The device of any one of claims 2 - 4, characterised in that the support member comprises a wall-tube passage component (12) for connection to the autoclave and a support tube (21) on the outer end of which is provided a fixing ring (27).

6. The device of claim 5, characterised in that the wall-tube passage component and the support tube are interconnected by a sealing flange (23).

7. The device of claim 3 or anyone of claims 4-6 when dependent on claim 3, characterised in that the tube (14) has an end ring (15) at its inner end.

9.

8.    The device of claim 7, <u>characterised in that</u> a sealing flange (16) is interposed between the  end ring (15) and the inner end of the support member and <u>in that</u> sealing rings (17,18) are provided between the end     ring and the sealing flange and packings (19) are provided between the sealing flange and support member.

9.    An autoclave provided with a device as set forth in any one of claims 2 - 7, the support member extending through and being connected to the wall of the autoclave.

European Patent
Office

**EUROPEAN SEARCH REPORT**

0117369

Application number

EP 83 30 8043

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 687 612 (R.R. ERNST) <br> * Column 1, lines 32-72; column 2 * | 1 | A 61 L 2/24 // <br> A 61 L 2/20 |
| | --- | | |
| A | US-A-3 936 270 (D.A. GUNTHER) <br> * Column 3, example * | 1 | |
| | --- | | |
| A | US-A-4 294 804 (W.J. BARAN) <br> * Column 1, lines 40-50 * | 1 | |
| | --- | | |
| A | DE-A-2 145 841 (G.D. SHAW) <br> * Claim 30 * | 1 | |
| | ----- | | |

|  |
|---|
| TECHNICAL FIELDS SEARCHED (Int. Cl. 3) |
| A 61 L 2/24 <br> A 61 L 2/20 |

*The present search report has been drawn up for all claims*

| Place of search | Date of completion of the search | Examiner |
|---|---|---|
| THE HAGUE | 09-04-1984 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82